# EUROPEAN PATENT SPECIFICATION

(11) **EP 4 311 517 B1**
(45) Date of publication and mention of the grant of the patent: **29.10.2025**
(21) Application number: 22195526.3
(22) Date of filing: 14.09.2022
(51) Int. Cl.: A61C 1/14, A61B 17/16

(54) **DENTAL HANDPIECE HAVING AN IMPROVED PUSH BUTTON GUIDANCE**
ZAHNÄRZTLICHES HANDSTÜCK MIT VERBESSERTER DRUCKTASTENFÜHRUNG
PIÈCE À MAIN DENTAIRE AYANT UN GUIDAGE PAR BOUTON POUSSOIR AMÉLIORÉ

(30) Priority: 27.07.2022 EP 22187241
(43) Date of publication of application: 31.01.2024
(73) Proprietor: Dentsply Sirona Inc., York, PA 17401 (US); Sirona Dental Systems GmbH, 64625 Bensheim (DE)
(72) Inventor: Ertugrul, Metin, 64625 Bensheim (DE)
(74) Representative: Taor, Simon Edward William

(56) References cited:
- EP-A1- 3 412 244

## Description

### TECHNICAL FIELD OF THE INVENTION

The present invention relates to dental handpieces, in particular to the push button guidance of a dental handpiece for actuating a clamping mechanism for a dental drill.

### BACKGROUND ART OF THE INVENTION

A dental handpiece is a handheld device for performing a variety of dental treatments which may require the use of various dental tools such as drills. The head of the dental handpiece is usually provided with a clamping mechanism such as a chuck that allows the dentist to change the dental tool in accordance with the intended dental treatment by means of a push button. The push button is movably held through a guide in the head of the dental handpiece.

Reference is made to EP0639354A2, EP 1733696A1, EP2959860A1 each disclosing a prior art dental handpiece.

EP 3 412 244 A1 discloses a dental handpiece comprising:a head;a clamping means which is rotatably arranged within a bearing inside the head, and adapted for releasably clamping a dental drill, wherein the bearing has an inner race fixed to the clamping means and an outer race fixed to a housing of the head; a driving means for rotating the clamping means about an axial direction of the dental drill;a push button which is arranged movably in the head along the axial direction and configured to actuate the clamping means to release the dental drill; and a biasing means to move the push button away from the clamping means; wherein a radial inner surface of the push button is arranged to contact and slide on a radial outer surface of a bearing cover which configures a guide for the moving push button, wherein the radial direction is perpendicular to the axial direction of the dental drill; and wherein a radial clearance is arranged between a radial inner surface of the housing and a radial outer surface of the push button.

There are certain design requirements that must be met by the dental handpiece to assure its proper operation. For example, any excessive heating of the push button at the contact area with the clamping mechanism must be prevented in as much as possible. For example, any excessive mechanical abrasion of the clamping jaws must be prevented in as much as possible. For example, the size of the head should be reduced as much as possible so that it can easily reach spatially limited places in the oral cavity. For example, the push button and the clamping mechanism must be operable in a reliable and easy manner.

### DISCLOSURE OF THE INVENTION

The objective of the present invention is to overcome one or more of the disadvantages of the prior art and provide a dental handpiece which is mechanically compact and easy to use.

This objective has been achieved through the dental handpiece as defined in claim 1.

The dental handpiece of the present invention comprises: a head; a clamping means which is rotatably arranged within a bearing inside the head, and adapted for releasably clamping a dental drill, wherein the bearing has an inner race fixed to the clamping means and an outer race fixed to a housing of the head; a driving means for rotating the clamping means about an axial direction of the dental drill; a push button which is arranged movably in the head along the axial direction and configured to actuate the clamping means to release the dental drill; and a biasing means to move the push button away from the clamping means. The radial inner surface of the push button is arranged to contact and slide on the radial outer surface of the outer race which configures a guide for the moving push button along the axial direction, wherein the radial direction is perpendicular to axial direction of the dental drill. A radial clearance is arranged between the radial inner surface of the housing and a radial outer surface of the push button.

Another major advantageous effect of the present invention is that the size of the head can be reduced. This can be explained as follows. Since the radial outer surface of the outer race which configures a guide for the moving push button along the axial direction, thereby the need for providing any separate guidance member can be avoided. This allows a compact mechanical configuration. The compact size of the head will also allow the dentist to easily reach spatially limited places in the oral cavity and to work more easily inside the oral cavity.

Another major advantageous effect of the present invention is that the push button is operable in a more reliable and easy manner. This can be explained as follows: Since the radial outer surface of the outer race which configures a guide for the moving push button along the axial direction, the motion can be property aligned with the bearing surfaces, and thereby with the drill of the clamping mechanism. This enables a good alignment between the moving parts.

In alternative embodiments of the present invention the push button can be provided as a single piece member or a two-piece member.

### BRIEF DESCRIPTION OF THE DRAWINGS

In the subsequent description, the present invention will be described in more detail by using exemplary embodiments and by referring to the drawings, wherein
Fig. 1 - is a schematic partial side view of a dental handpiece according to an embodiment of the invention;
Fig. 2a - is a schematic partial vertical cross-sectional enlarged view of the head of the dental handpiece in Fig 1, taken along the line I-I;
Fig. 2b - is a schematic partial cross-sectional enlarged view of the head of the dental handpiece in Fig 1, taken along the line **I-I** in the actuated state;
Fig. 2c - is a schematic partial cross-sectional view of the head of the dental handpiece of Fig.1, taken along the line II-II;
Fig. 2d - is a schematic partial cross-sectional enlarged view of the head of a dental handpiece according to an alternative embodiment of the invention, taken along the line **II-II** in Fig 1;
Fig. 3 - is a schematic partial cross-sectional enlarged view of the clamping mechanism inside the bearings of the dental handpiece in Fig 1, taken along the line I-I;
Fig. 4a - is a schematic partial cross-sectional enlarged view of the clamping mechanism of Fig. 3 without the bearings;
Fig. 4b - is a schematic partial cross-sectional view of the clamping mechanism of Fig 4a, in the actuated state;
Fig. 5a - is a schematic side view of the chuck of the dental handpiece in Fig 1;
Fig. 5b - is a schematic perspective view of the chuck of the dental handpiece in Fig 1;

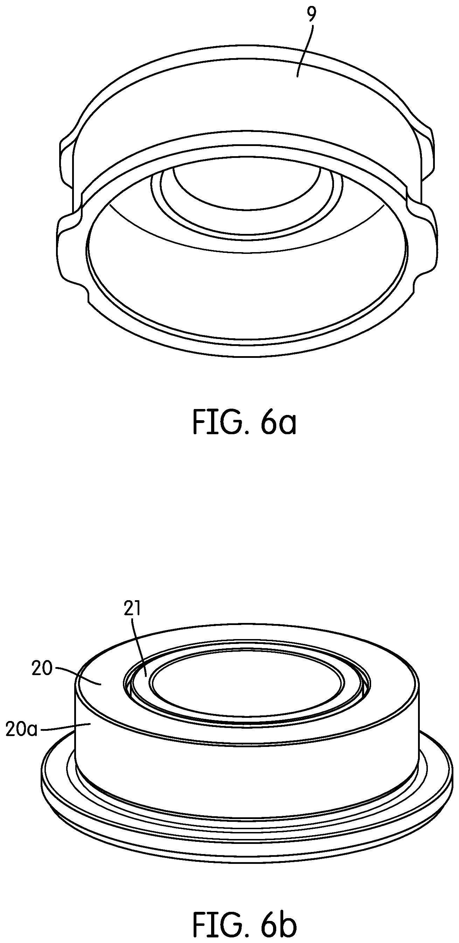

Fig. 6a - is a schematic perspective view of the flange collar (hat-shaped) of the dental handpiece in Fig 1;
Fig. 6b - is a schematic perspective view of the outer race and the inner race of the dental handpiece in Fig 1;

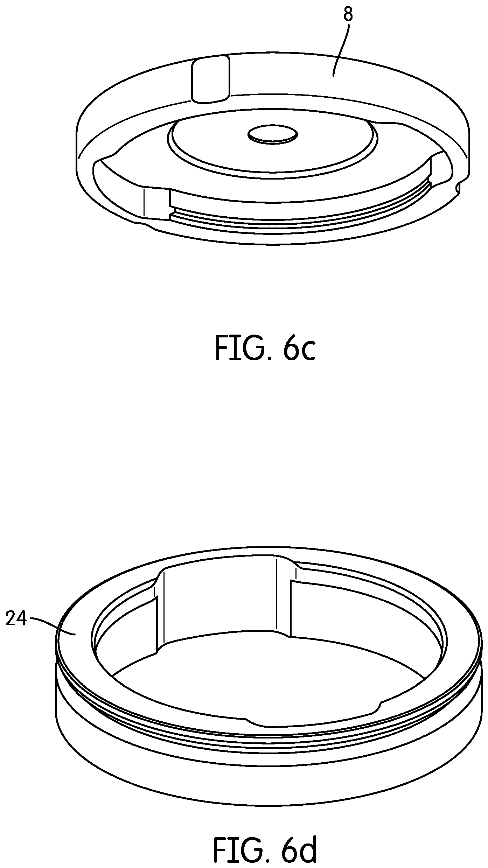

Fig. 6c - is a schematic perspective view of the cap of the dental handpiece in Fig 1;
Fig. 6d - is a schematic perspective view of the screw ring of the dental handpiece in Fig 1;
Fig. 7a - is a schematic vertical cross sectional view of the two-piece push button of the dental handpiece in Fig 2c;
Fig. 7b - is a schematic perspective view of the two-piece push button in Fig 7a;
Fig. 7c - is a schematic vertical cross sectional view of the single-piece push button of the dental handpiece in Fig 2d;
Fig. 7d - is a schematic perspective view of the single-piece push button in Fig 7c;

The reference numbers shown in the drawings denote the elements as listed below and will be referred to in the subsequent description of the exemplary embodiments.
- 1.: Dental handpiece
- 2.: Dental drill (burr)
- 3.: Body
- 4.: Head
- 5.: Housing
5a. Gap
5b. Radial inner surface
- 6.: Internal Thread
- 7.: Pushbutton
7a. Radial inner surface
7b. Radial outer surface
- 8.: Cap
- 9.: Flange collar (hat-shaped)
9a. Claw
9b. Air Gap (thermal insulation)
- 9': Cap
- 10.: Contact surface (point like)
- 11.: Spring (cone shaped)
- 12.: Guide
- 13.: Clamping means
- 14.: Ball
- 15.: Hollow shaft
- 16.: Gear
- 17.: Opening
- 18.: Stop Edge
- 19.: 19' Bearing
- 20.: Outer race
20a. Radial outer surface
- 21.: Inner race
- 22.: Balls
- 23.: Retainer
- 24.: Screw ring
- 25.: Nose
- 26.: Chuck
- 27.: Arm
27a. Contact point
- 28.: Cut-out
- 29.: Large diameter portion
- 30.: Small diameter portion
- 31.: Contact edge (Burr stop)
- 32.: Driving shaft
- 33.: Bearing
- 34.: Outer race
- 35.: Inner race
- 36.: Balls
- 37.: Retainer
- 38.: Crown gear

A: Axial Direction
R: Radial Direction
S: Radial Clearance
F: Actuation Force

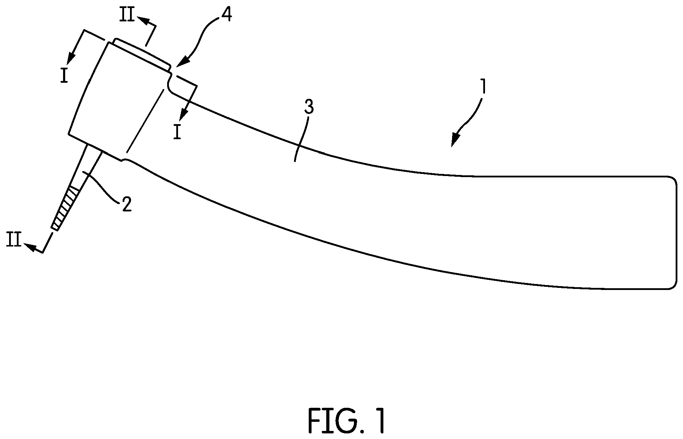

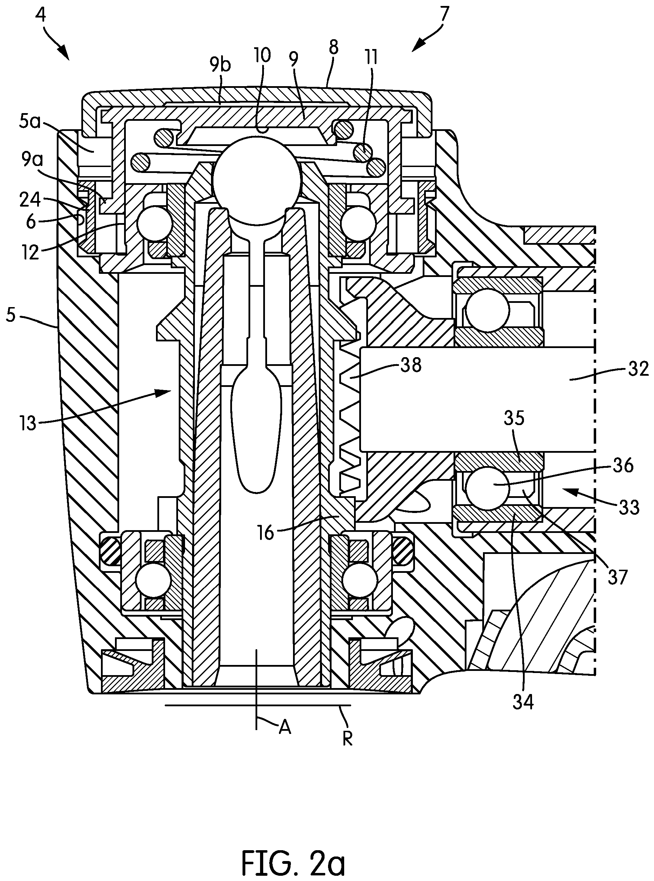

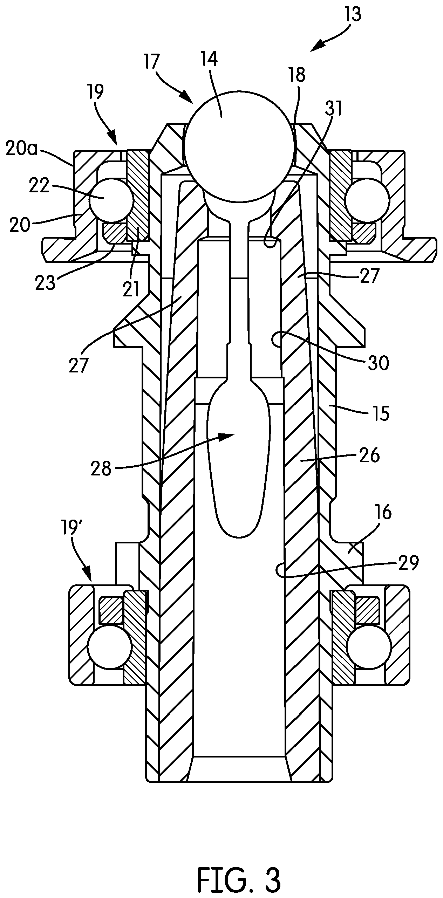

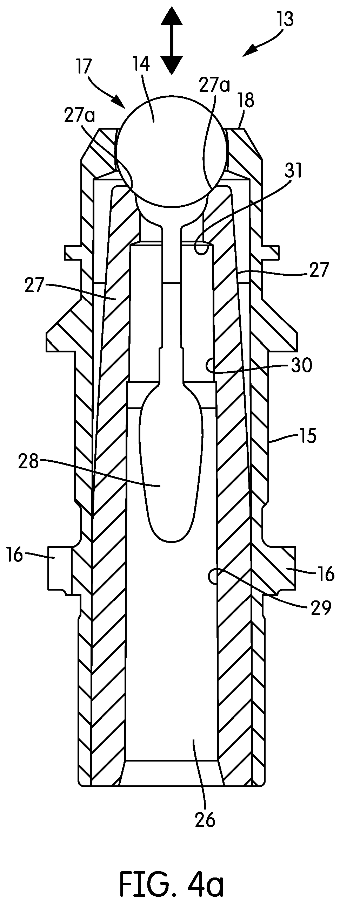

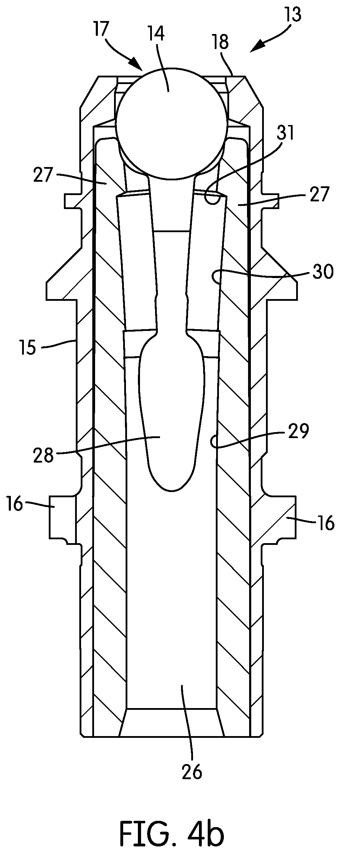

Fig. 1 shows a dental handpiece (1) according to an embodiment of the invention. As shown in Fig. 1, dental handpiece (1) has a body (3) and a head (4). A dental drill (2) is detachably attached to the head (4). Fig. 2a shows an enlarged cross-sectional view of the head (4). As shown in Fig. 2a, the head (4) has a housing (5) which accommodates a clamping means (13). As shown in Fig. 4a the clamping means (13) has a chuck (26) with resilient arms (27) for releasably clamping the dental drill (2). A hollow shaft (15) is rotatably arranged inside the head (4). The chuck (26) is fixed into the hollow shaft (15) to rotate with same. The dental handpiece (1) has a driving means such as a motor (not shown) for rotating the hollow shaft (15). The driving means is coupled to a driving shaft (32) as shown in Fig. 2a, that is rotatably held through a bearing (33) having an outer race (34), an inner race (35), multiple balls (36), and a retainer (37). The end of the driving shaft (32) has a crown gear (38) that meshes with a gear (16) on the hollow shaft (15). As shown in Fig. 3 the hollow shaft (15) is rotatably held through a couple of bearings (19; 19') mounted into the housing (5). Each bearing (19;19') has an outer race (20) and inner race (21), multiple balls (22) and a retainer (23).

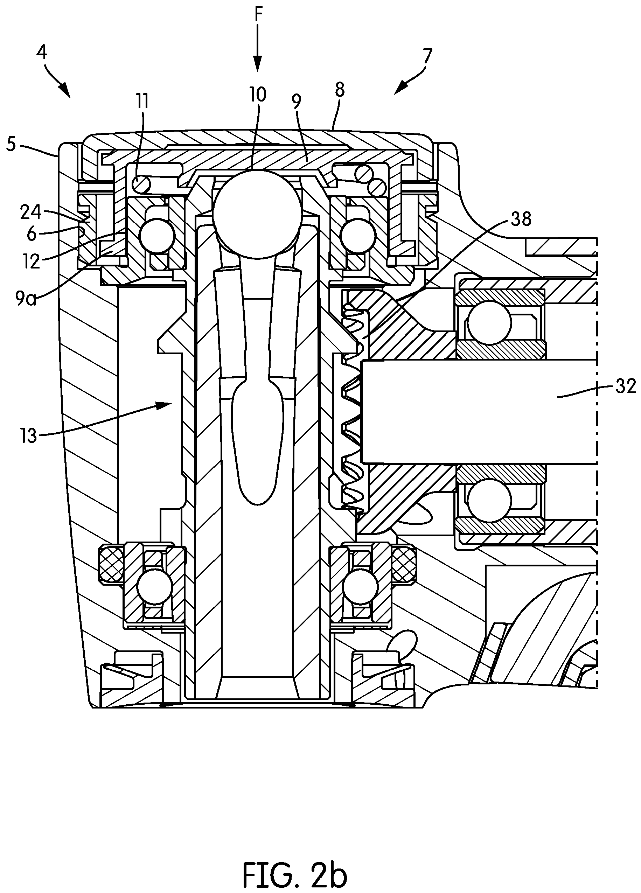

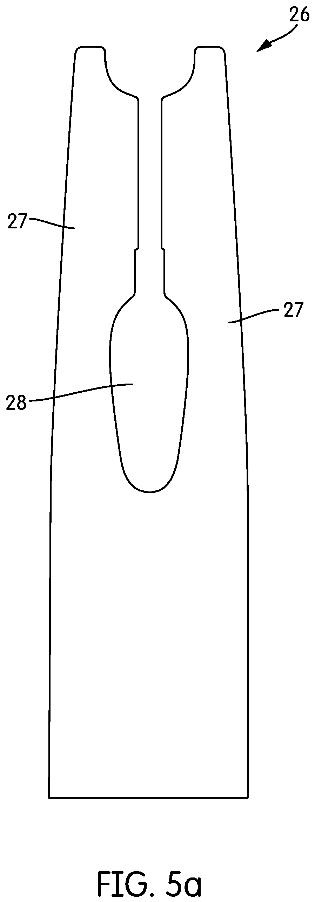

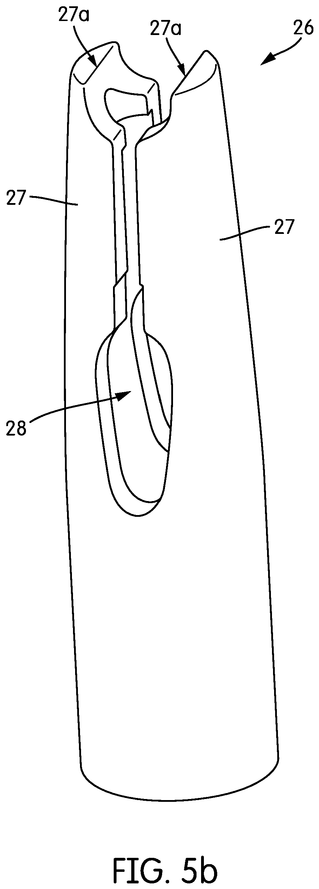

As shown in Fig. 3, the clamping means (13) has a ball (14), preferably made of ceramic material, which is axially movably arranged inside the rotatable hollow shaft (15) and in direct contact with the resilient arms (27) of the chuck (26). The ball (14) partly protrudes through an opening (17) in the upper end of the hollow shaft (15). The size of the opening (17) is smaller than the size of the ball (14) so that it is retained partly within the hollow shaft (15). As shown in Fig. 3 the opening (17) has an annular stop edge (18) which restricts the outwards movement of that ball (14). As shown in Fig. 2a and Fig. 2b the dental handpiece (1) has a push button (7) which is arranged in the head (4) and configured to contact, when pressed by a user, the partly protruding ball (14) and move the ball (14) downwardly in between the resilient arms (27) to flex the resilient arms (27) radially outwards and release the dental drill (2). As shown in Fig. 5a the chuck (26) has two diametrically opposed cut-outs (28) in its upper portion to form the two resilient arms (27). As shown in Fig. 5b, a linear semi cylindrical channel is formed into the upper portion of the chuck (26) whose edges form two diametrically opposed contact points (27a) to contact the ball (14). As shown in Fig. 3, the chuck (26) has a large diameter portion (29) and a small diameter portion (30). When the resilient arms (27) are retracted the dental drill (2) can be manually inserted only until the upper end of the large diameter portion (29). When the resilient arms (27) are flexed through the ball (14), the dental drill (2) can be further inserted into the small diameter portion (30) until it abuts against the contact edge (31) (Burr stop). Once the push button (7) is released, the resilient arms (27) are retracted, and the dental drill (2) is clamped in the small diameter portion (30) between the resilient arms (27). The resilient arms (27) also bias the ball (14) inside the rotatable hollow shaft (15) towards the opening (17). As shown in Fig. 2a, the pushbutton (7) has a cap (8), and a hat-shaped flange collar (9) arranged inside the cap (8) and fixed thereto. The hat-shaped flange collar (9) has a point like contact surface (10), preferably planar, for the ball (14). The pushbutton (7) has a biasing means such a spring (11), preferably a cone shaped spring (11) to move the push button (7) away and detach it from the ball (14). As shown in Fig. 2a, the point like contact surface (10) is not in contact with the ball (14) when the push button (7) is released. As shown in Fig. 2b, the contact surface (10) comes in point contact with the upper side of the ball (14) when the push button (7) is pressed downwards.

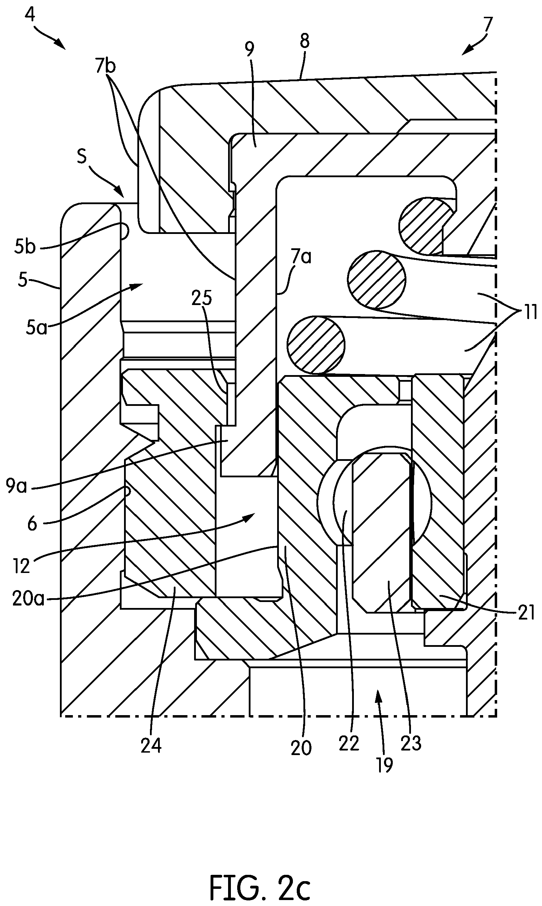

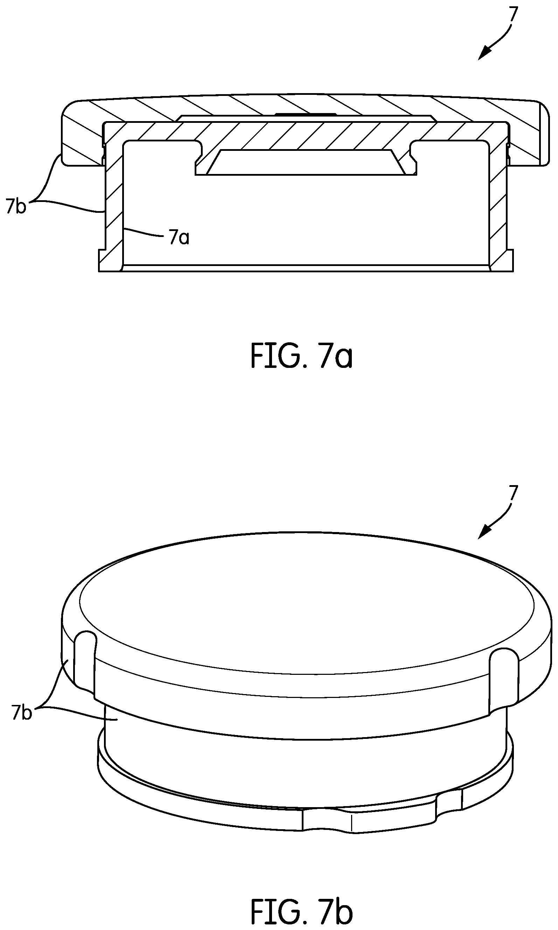

Fig. 2c shows an enlarged view of the two-piece pushbutton (7) in Fig. 2a which is configured by the cap (8) and the hat-shaped Flange collar (9). Fig. 7a and Fig. 7b, show the two-piece push button (7) in more detail.

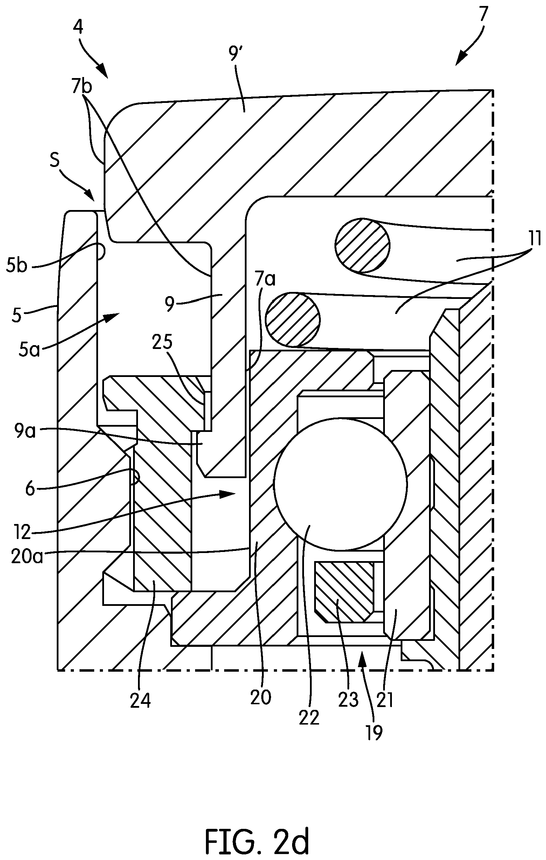

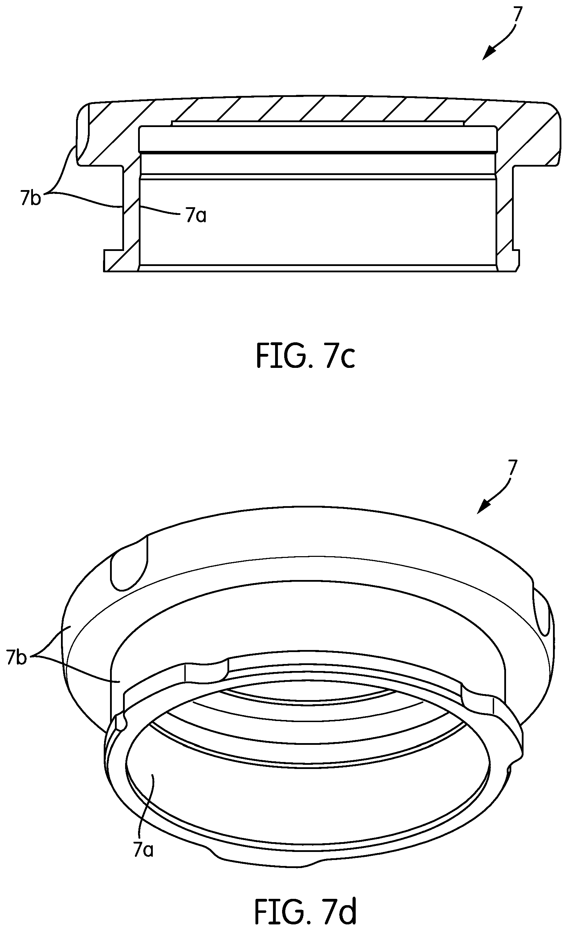

Fig. 2d shows an alternative embodiment, wherein the cap (9') is provided as a single piece instead of two separate pieces. All other features remain the same. Thereby the number of pieces can be further reduced. Fig. 7c and 7d, show the single-piece push button (7) in more detail.

According to the alternative embodiments as shown in Fig. 2c and 2d, a radial inner surface (7a) of the push button (7) is arranged to contact and slide on a radial outer surface (20a) of the outer race (20) which configures a guide (12) for the moving push button (7). The radial direction is perpendicular to the axial direction (A) of the dental drill (2). A radial clearance (s) is arranged between a radial inner surface (5b) of the housing (5) and a radial outer surface (7b) of the push button (7).

As shown in Fig. 2a, the housing (5) has an internal thread (6) which receives a screw ring (24). The mounting flange of the upper bearing (19) is fixed to its mounting position through the screw ring (24). The screw ring (24) has a nose (25) which can be seen in Fig. 2d but not in Fig.2a. The cross-sectional views respectively in Fig. 2a and Fig. 2d are rotated about 90 degrees with respect to each other. The cross-section in Fig. 2a is along the line I-I as shown in Fig. 1 and the cross section in Fig. 2d is along the line II-II as shown in Fig. 1. As shown in Fig. 2d, the nose (25) extends radially inwards and can abut from below against the claw (9a) of the hat-shaped flange collar (9) which extends radially outwards and prevents the push button (7) from disengaging from the housing (5). The hat-shaped flange collar (9) can be provided separately from or integrally with the cap (8;9'). In the former case an air gap (9b) is preferably formed underneath the cap (8) to improve thermal insulation as shown in Fig. 2a. Alternatively the air gap (9b) may be filled with a material that is a poor heat-conductor.

As shown in Fig. 2a and Fig.2d, the inner surface of the hat-shaped flange collar (9) slides on the outer surface of outer race (20) while being in contact with the latter which configures a guide (12) for the push button (7) movement. The nose (25) of the screw ring (24) is arranged above the claw (9a) of the hat-shaped flange collar (9). The hat-shaped flange collar (9) does not contact the inner radial surface of the screw ring (24). The housing (5) has a gap (5a) directly above the screw ring (24) and the nose (25), which allows the cap (8;9') to move upwards or downwards during the clamping/releasing operation. The screw ring (24) is entirely embedded/recessed inside the housing (5) and does not project outside the head (4). Thereby, the height of the head (4) can be reduced. Thereby also the diameter of the cap (8,9') can be increased so that the actuating pressure on the finger is reduced. The outer cylindrical surface of the cap (8;9') does not contact the inner surface of the housing (5) during its entire movement thanks to the sufficient radial clearance (S). Thus, guide (12) serves as a guiding contact surface for the up/down movement of the cap (8;9'). Through the above described configuration, the height and diameter of the head (4) can be further reduced.

## Claims

1. A dental handpiece (1) comprising:
a head (4);
a clamping means (13) which is rotatably arranged within a bearing (19) inside the head (4), and adapted for releasably clamping a dental drill (2), wherein the bearing (19) has an inner race (21) fixed to the clamping means (13) and an outer race (21) fixed to a housing (5) of the head (4);
a driving means for rotating the clamping means (13) about an axial direction of the dental drill (2);
a push button (7) which is arranged movably in the head (4) along the axial direction and configured to actuate the clamping means (13) to release the dental drill (2); and
a biasing means to move the push button (7) away from the clamping means (13);
a radial inner surface (7a) of the push button (7) is arranged to contact and slide on a radial outer surface (20a) of the outer race (20) which configures a guide (12) for the moving push button (7), wherein the radial direction is perpendicular to the axial direction of the dental drill (2); and wherein a radial clearance (S) is arranged between a radial inner surface (5b) of the housing (5) and a radial outer surface (7b) of the push button (7).

## Patentansprüche

1. Zahnärztliches Handstück (1), umfassend:
einen Kopf (4);
ein Klemmmittel (13), das drehbar innerhalb eines Lagers (19) innerhalb des Kopfes (4) angeordnet ist und zum lösbaren Klemmen eines zahnärztlichen Bohrers (2) ausgelegt ist, wobei das Lager (19) einen Innenring (21), der an dem Klemmmittel (13) befestigt ist, und einen Außenring (21), der an einem Gehäuse (5) des Kopfes (4) befestigt ist, aufweist;
ein Antriebsmittel zum Drehen des Klemmmittels (13) um eine axiale Richtung des zahnärztlichen Bohrers (2);
eine Drucktaste (7), die entlang der axialen Richtung bewegbar in dem Kopf (4) angeordnet und konfiguriert ist, um das Klemmmittel (13) zu betätigen, um den zahnärztlichen Bohrer (2) freizugeben; und
ein Vorspannmittel, um die Drucktaste (7) weg von dem Klemmmittel (13) zu bewegen;
wobei eine radiale Innenfläche (7a) der Drucktaste (7) angeordnet ist, um eine radiale Außenfläche (20a) des Außenrings (20), die eine Führung (12) für die bewegliche Drucktaste (7) konfiguriert, zu kontaktieren und daran zu gleiten, wobei die radiale Richtung senkrecht zu der axialen Richtung des zahnärztlichen Bohrers (2) ist; und wobei ein radialer Freiraum (S) zwischen einer radialen Innenfläche (5b) des Gehäuses (5) und einer radialen Außenfläche (7b) der Drucktaste (7) angeordnet ist.

## Revendications

1. Pièce à main dentaire (1) comprenant :
une tête (4) ;
un moyen de serrage (13) qui est agencé de manière rotative au sein d'un palier (19) à l'intérieur de la tête (4), et adapté pour serrer de manière libérable un foret dentaire (2), ledit palier (19) comportant une bague interne (21) fixée au moyen de serrage (13) et une bague externe (21) fixée à un boîtier (5) de la tête (4) ;
un moyen d'entraînement destiné à faire tourner le moyen de serrage (13) autour d'une direction axiale du foret dentaire (2) ;
un bouton poussoir (7) qui est agencé de manière mobile dans la tête (4) le long de la direction axiale et conçu pour actionner le moyen de serrage (13) de manière à libérer le foret dentaire (2) ; et
un moyen de sollicitation destiné à éloigner le bouton poussoir (7) du moyen de serrage (13) ;
une surface interne radiale (7a) du bouton poussoir (7) qui est agencée pour entrer en contact et glisser sur une surface externe radiale (20a) de la bague externe (20) qui configure un guide (12) pour le bouton poussoir mobile (7), ladite direction radiale étant perpendiculaire à la direction axiale du foret dentaire (2) ; et
un jeu radial (S) étant agencé entre une surface interne radiale (5b) du boîtier (5) et une surface externe radiale (7b) du bouton poussoir (7).
